# EUROPEAN PATENT APPLICATION

(11) **EP 4 624 575 A1**
(43) Date of publication of application: **01.10.2025**
(21) Application number: 25158318.3
(22) Date of filing: 17.02.2025
(51) Int. Cl.: C12N 15/113, A61K 31/7088

(54) **USE OF ANTISENSE OLIGONUCLEOTIDE IN PREPARATION OF DRUG FOR TREATING DISEASE CAUSED BY ABNORMAL THYROID**

(30) Priority: 15.03.2024 CN 202410298113
(71) Applicant: Hangzhou Tianlong Pharmaceutical Co., Ltd., Hangzhou, Zhejiang 310009 (CN)
(72) Inventor: SONG, Gengshen, Hangzhou, Zhejiang 310009 (CN); ZHOU, Yuting, Hangzhou, Zhejiang 310009 (CN)
(74) Representative: Ricker, Mathias

(57) **Abstract**

The present disclosure relates to the field of gene therapies of thyroid-associated ophthalmopathy (TAO) and, in particular, to a use of an antisense oligonucleotide in preparation of a drug for treating a disease caused by an abnormal thyroid. Experimental results of the present application indicate that an antisense oligonucleotide S-ASODN-1 with a particular sequence targeting an insulin-like growth factor 1 receptor (IGF-1R) can effectively reduce concentrations of the IGF-1R and an anti-thyroid peroxidase antibody (TPO-Ab), thereby improving a level of thyroid-stimulating hormone (TSH) for treating thyroid-associated ophthalmopathy.

## Description

### TECHNICAL FIELD

The present application belongs to the field of gene therapies of thyroid-associated ophthalmopathy and, in particular, relates to a use of a full thioantisense oligonucleotide taking an insulin-like growth factor 1 receptor (IGF-1R) gene as a target or a composition thereof in a thyroid-associated ophthalmopathy model and in a treatment of thyroid-associated ophthalmopathy.

### BACKGROUND

Thyroid-associated ophthalmopathy (TAO) (also referred to as thyroid eye disease (TED), Graves' ophthalmopathy or orbitopathy (GO), thyrotoxic proptosis, thyroid dysfunction ophthalmopathy and several other terms) is orbitopathy related to an abnormal thyroid function and ranks the first in incidences of periorbital diseases of adult humans. TAO is also the most common extrathyroidal manifestation of Graves' disease (GD) with a morbidity accounting for 25% to 40% of GD and may also be seen in 2% patients with chronic lymphocytic thyroiditis, minority patients with thyroid function hypothyroidism and people with normal thyroid functions. According to an article from the Chinese Center for Disease Control and Prevention, a total morbidity of GD in people is about 1%, and a morbidity rate in women is far higher than that in men. An estimated TAO morbidity is between 0.25% and 0.4%.

TAO is an organ-specific autoimmune orbital inflammatory disease associated with the abnormal thyroid function, and a human body attacks orbital fibroblasts overexpressing IGF-1R in the human body so that muscles and adipose tissues behind eyes are inflamed, thereby causing the eyes to push forward and bulge outward. This irreversible damage after long-term accumulation can even cause blindness.

TAO is classified into two types. Generally, active TAO lasting 1 to 3 years is characterized by a persistent autoimmune/inflammatory response in orbital soft tissues. Active TAO causes the expansion and remodeling of ocular soft tissues. The autoimmune/inflammatory response of active TAO spontaneously retreats and the condition transitions to inactive TAO. Inactive TAO is a term for describing a long-term/permanent sequela of active TAO. A cause of TAO is unknown. TAO is generally associated with Graves' hyperthyroidism but may also appear as another autoimmune condition to affect a thyroid and generate pathologies in orbits and periorbital tissues and rarely generate pathologies in pretibial skins (pretibial myxedema) or fingers (thyroid acropachy). TAO is an autoimmune orbital disease. Primarily, the orbits and the periocular soft tissues are affected, and secondly, the eyeballs and the vision are affected. In TAO, the eyeballs are forced to move forward (bulging) away from eye sockets of the eyeballs due to the inflammation and expansion of the orbital soft tissues (mainly eye muscles and adipose)-a phenomenon called protopsis or exophthalmos. Although most cases of TAO do not cause vision loss, this condition may cause vision-threatening exposure keratopathy, troublesome diplopia/double vision and compressive dysthyroid optic neuropathy. TAO may appear before, simultaneously with or after dysthyroid systemic complications. Ocular clinical manifestations of TAO, which include upper eyelid retraction, eyelid lag, swelling, redness (erythema), conjunctivitis and bulging eyes (proptosis or exophthalmos), chemosis, periorbital edema and altered eye movement, have significant functional, social and cosmetic consequences. Many signs and symptoms of TAO (including proptosis and bloodshot eyes) are caused by the expansion of orbital adipose tissues and periocular muscles. An increase in an adipose tissue volume is due in part to the development of new adipocytes (adipogenesis) in orbital adipose. The accumulation of a hydrophilic glycosaminoglycan (mainly hyaluronic acid (HA)) in the perimuscular connective tissues between the orbital adipose tissues and the extraocular muscle fibers further expands an adipose compartment and increases extraocular muscles. HA is generated by fibroblasts retained in the orbital adipose and the extraocular muscles, and *in vitro* synthesis of HA is stimulated by several cytokines and growth factors (including IL-1β, interferon-γ, a platelet-derived growth factor and thyroid-stimulating hormone (TSH)).

For a long time, no optimal treatment measure has yet been provided for medium and severe active TAO, and a first-line treatment solution is a glucocorticoid intravenous shock therapy with the problems such as not ideally improved proptosis and the existence of hormone-related systemic side effects and a still relatively large unmet clinical requirement. A second-line treatment includes another shock with a hormone, combined orbital radiotherapy or other immunomodulators. Biological agents such as teprotumumab, tocilizumab and rituximab are also recommended by the EUGOGO (European Group on Graves' Orbitopathy) Guidelines, the Chinese Guidelines on the Clinical Diagnosis and Treatment of Thyroid-associated Ophthalmopathy (2022) and the Consensus on Thyroid-associated Ophthalmopathy by the American Thyroid Association and the European Thyroid Association as a second-line treatment solution for medium and severe active TAO. In particular, for TAO combined with significant proptosis, teprotumumab may be used as a first choice.

Invasive CD34+ fibrocytes derived from a myeloid monocyte lineage enter from a circulatory system and lay a foundation for a disease. Autoantigens expressed and presented by the invasive CD34+ fibrocytes may be similar to antigens found in the thyroid. Low-level TSHR (thyroid-stimulating hormone receptor), thyroglobulin and other thyroid antigens are included. The fibrocytes present the antigens to antigen-specific T cells that in turn support IgG1 generation by the B cells. These fibrocytes are differentiated into CD34+ fibroblasts. According to regulatory signals received by these fibroblasts, these fibroblasts are further differentiated into myofibroblasts and adipocytes. Fibroblasts resident in a portion of orbits are CD34-. After entering the orbits, the CD34+ fibroblasts encounter the resident CD34-fibroblasts. When these cells are activated, these cells generate a number of proinflammatory and anti-inflammatory factors including cytokines (including interleukins-1β, -6, -8, -10, -12 and -16), tumor necrosis factors-α, chemokines referred to as "regulated upon activation, normal T cell expressed and secreted" (RANTES), CXCL12 and CD40 ligands (CD40LCD154). These cytokines can act locally on almost all the resident cells in the orbits. Types of almost all the invasive cells and the resident cells are IGF-1R. Therefore, a treatment method based on the inhibition of IGF-1R may cover all these cells. Fibroblasts activated by the cytokines synthesize HA and other glycosaminoglycans, and these substances can enlarge an orbital tissue volume and can cause proptosis.

In addition, an autoantibody of a thyroid-stimulating hormone receptor (TSHR) is used as a TSHR agonist for inducing the excessive secretion of thyroid hormone, causing that the thyroid is not controlled by pituitary gland. The autoantibody of the TSHR is also a basis of Graves' ophthalmopathy and pretibial myxedema. A synergistic interaction between IGF-1R and the autoantibody of the TSHR leads to retro-orbital tissue expansion and inflammation.

Some drugs entering a clinical stage at present for treating TAO are introduced in Table 1.

**Table 1 TAO drugs under development**

| **No.** | **Drug Name** | **Target** | **Mechanism of Action** | **Research and Development Institution** | **Clinical Stage** |
|---|---|---|---|---|---|
| **1** | secukinumab | IL-17A | anti-IL-17A monoclonal antibody | Novartis | phase I clinical trial |
| **2** | tocilizumab | IL-6R | anti-IL-6R monoclonal antibody | Chugai Pharmaceutical | phase II clinical trial |
| **3** | satralizumab | IL-6R | anti-IL-6R monoclonal antibody | Chugai Pharmaceutical | phase III clinical trial |
| **4** | vunakizumab | IL-17A | anti-IL-17A monoclonal antibody | Jiangsu Hengrui Pharmaceuticals Co., Ltd. | phase II clinical trial |
| **5** | batoclimab | FcRn | anti-FcRn monoclonal antibody | CSPC Pharmaceutical Group; HanAll Biopharma; Immunovant; Harbour BioMed | phase III clinical trial |
| **6** | TOUR006 | IL-6 | anti-IL-6-antibody | Tourmaline Bio | phase II clinical trial |
| **7** | LASN01 | IL-11RA | anti-IL-11RA monoclonal antibody | CSL; Lassen Therapeutics | phase I clinical trial |

Insulin-like growth factors (IGFs), which are also referred to as growth regulators, include IGF-1 and IGF-2. Only by binding to receptors IGF-1Rs of these growth factors can these growth factors exert biological effects. The IGF-1Rs belong to a receptor tyrosine kinase family and are located on cell membranes. After binding to the IGFs, the IGF-1Rs are dimerized so that tyrosine domains can approach each other and trigger autophosphorylation, thereby subsequently activating signaling pathways such as RAS-RAF-MAPK and PI3K-PKB/AKT that are associated with cell proliferation in cells. The activation of the IGF-1Rs is crucial for stimulating the growth and survival of tumor cells.

Some indications of drugs targeting the IGF-1Rs under development at present are shown in Table 2, and TAO is not involved.

**Table 2 Drugs targeting IGF-1Rs under development**

| **No.** | **Drug Name** | **Stage (Global)** | **Target** | **Indication** |
|---|---|---|---|---|
| **1** | Conteltinib | phase III clinical trial | ALK, FAK, PYK2, IGF-2R | non-small-cell lung carcinoma |
| **2** | Xentuzumab | phase II clinical trial | IGF-1, IGF-3 | breast cancer |
| | | phase II clinical trial | | castration-resistant prostate cancer |
| | | phase I clinical trial | | non-small-cell lung carcinoma |
| **3** | WOKVAC | phase II clinical trial | IGF-1R, IGFBP2 , HER3 | breast cancer |
| **4** | IGV-002 | phase II clinical trial | IGF-1R | glioblastoma |
| **5** | lonigutamab ugodotin | phase I/II clinical trial | IGF-1R | solid tumor |
| **6** | IGF-MTX | phase I/II clinical trial | IGF-1R | myelodysplastic syndrome |
| **7** | [111In]-FPI-1548 | phase I clinical trial | IGF-1R | solid tumor |
| | | phase I clinical trial | | radionuclide imaging |
| **8** | [225Ac]-FPI-1435 | phase I clinical trial | IGF-1R | solid tumor |
| **9** | 68Ga-NODAGA-ZIG F-1R:4:41 | phase I clinical trial | IGF-1R | PET imaging |
| | | phase I clinical trial | | cancer |
| **10** | KT-A833 | before clinical trial | IGF-2 | type 1 diabetes |
| **11** | [11C]GSK1838706A | before clinical trial | IGF-1R | PET imaging |

Theoretically, an IGF-1R inhibitor can prevent the IGF-1R from binding to the TSHR and block downstream signaling mediated by a TSHR/IGF-1R complex, thereby improving TAO. In recent years, newly developed GIP-1R monoclonal antibodies have mostly focused on TAO. In the field of TAO, teprotumumab is the only drug on the market.

An antisense oligodeoxyribonucleotide (ASODN) is a type of artificially synthesized oligonucleotide fragment that is mostly 15 to 30 nucleotides in length and mainly interferes with the transcription and translation of a target gene through a principle of complementary base pairing, thereby achieving a targeted therapy of the gene. The ASODN has the advantages of abundant candidate targets, a rationally designed direction, efficient effects in vivo and in vitro and large-scale artificial synthesis and is considered to be a gene therapy drug with great potential. In recent years, with breakthroughs in nucleotide chemical modification technology and delivery technology, ASODN drug research and development has set off a new wave, and some well-known pharmaceutical companies at home and abroad have used antisense drugs as one of the key directions for new drug research and development. At present, nine antisense oligonucleotide drugs have been on the market (Table 3), and TAO is not involved.

**Table 3 Antisense oligonucleotide drugs on the market**

| **Drug Name** | **Year on the Market** | **Indication** |
|---|---|---|
| Fomivirsen | 1998 | for treating cytomegalovirus(CMV) retinitis complicated in patients with AIDS |
| Mipomersen | 2013 | for patients with homozygous familial hypercholesterolemia (HoFH) |
| Eteplirsen | 2016 | for treating Duchenne muscular dystrophy (DMD) |
| Nusinersen | 2016 | for treating spinal muscular atrophy (SMA) |
| Inotersen | 2018 | for treating polyneuropathy in patients with hereditary transthyretin amyloidosis (hATTR) |
| Volanesorsen | 2019 | for an adjunctive therapy for adult patients with familial chylomicronaemia syndrome (FCS) beyond controlling diet |
| Golodirsen | 2019 | for treating patients with Duchenne muscular dystrophy (DMD) |
| Viltolarsen | 2020 | for treating Duchenne muscular dystrophy (DMD) |
| Amondys 45 | 2021 | for treating patients with gene mutation exon 45 skipping Duchenne muscular dystrophy (DMD) |

Therefore, providing an antisense oligonucleotide for treating TAO has important practical significance.

### SUMMARY

In view of this, an antisense oligonucleotide S-ASODN-1 having a unique sequence and targeting IGF-1R is designed in the present application to treat TAO.

The present application discovered for the first time that an antisense oligonucleotide S-ASODN-1 with a particular sequence targeting an IGF-1R can effectively reduce concentrations of the IGF-1R and an anti-thyroid peroxidase antibody (TPO-Ab), thereby improving a level of TSH for treating TAO. In essence, among existing drugs for treating TAO that have been approved or have entered a clinical stage at present, there is no antisense oligonucleotide. Moreover, not all antisense oligonucleotides targeting IGF-1R and having any sequence can treat TAO. For example, other antisense oligonucleotide sequences S-ASODN-2, S-ASODN-3, S-ASODN-4 and S-ASODN-5 in the present application cannot effectively regulate TPO-Ab and TSH levels and cannot be used for treating TAO.

The present application provides a use of a therapeutically effective amount of a thioantisense oligonucleotide targeting an IGF-1R gene or a composition thereof in preparation of a drug for treating a disease caused by an abnormal thyroid function, where the thioantisense oligonucleotide targeting the IGF-1R gene has:
(I) a nucleotide sequence shown in SEQ ID No. 1; or
(II) a nucleotide sequence obtained after one or more nucleotide sequences in the nucleotide sequence shown in (I) are substituted or deleted or one or more nucleotide sequences are added to the nucleotide sequence shown in (I) and having the same or similar function as the nucleotide sequence shown in (I); or
(III) a nucleotide sequence having at least 80% nucleotide identity to the nucleotide sequence shown in (I) or (II);

wherein the thioantisense oligonucleotide refers to phosphorothioate modified antisense oligonucleotide;
preferably, the thioantisense oligonucleotide targeting the IGF-1R gene has a nucleotide sequence having 85%, 90%, 95%, 96%, 97%, 98%, 99% or more nucleotide identity to the nucleotide sequence shown in (I) or (II).

In some preferred embodiments, the sequence of the thioantisense oligonucleotide targeting the IGF-1R gene is a nucleotide sequence including more than 80% nucleotide identity to a 5'-TCCTCCGGAGCCAGACTTCA-3' (SEQ ID NO:1) sequence, preferably, the nucleotide sequence has 85%, 90%, 95%, 96%, 97%, 98%, 99% or more nucleotide identity, and more preferably, the sequence of the thioantisense oligonucleotide targeting the IGF-1R gene is 5'-TCCTCCGGAGCCAGACTTCA-3' (SEQ ID NO:1).

In some preferred embodiments, the disease caused by the abnormal thyroid function includes TAO.

In some preferred embodiments, the thioantisense oligonucleotide targeting the IGF-1R gene further has other chemical modifications.

In some preferred embodiments, the other chemical modifications are selected from one or more of a locked nucleic acid modification, a methoxyethyl modification at a 2 position and an oxomethyl modification at a 2 position. The 2 position is the 2' position of the ribose ring.

In some preferred embodiments, the composition further includes at least one additional active agent.

In some preferred embodiments, the at least one additional active agent is a therapeutic agent.

In some preferred embodiments, the therapeutic agent is selected from one or more of a corticosteroid, a biological agent and a traditional immunosuppressant.

In some preferred embodiments, the corticosteroid is selected from a glucocorticoid.

In some preferred embodiments, using an intravenous glucocorticoid (ivGC) and an oral glucocorticoid for treating patients with medium to severe active TAO is recorded in Patent 202180081949.7.

In some preferred embodiments, the biological agent is selected from one or more of a CD20+B cell inhibitor, an IL-6R antibody, an IL-17A antagonist, an FcRn antagonist, an IL-11R blocking antibody, an anti-TNFα antibody and a TSHR inhibitor.

In some preferred embodiments, the traditional immunosuppressant is selected from one or more of mycophenolate mofetil, ciclosporin, methotrexate and azathioprine.

Chinese Guidelines on the Diagnosis and Treatment of Thyroid-Associated Ophthalmopathy (2022), Chinese Journal of Ophthalmology, September 2022, Vol. 58, No. 9

In some preferred embodiments, the therapeutic agent is selected from one or more of secukinumab, tocilizumab, satralizumab, vunakizumab, batoclimab, TOUR006, LASN01, infliximab, rituximab and selenium.

In some preferred embodiments, a pharmaceutical composition for treating TAO further including a corticosteroid, rituximab or other anti-CD20 antibodies, tocilizumab or other anti-IL-6 antibodies, or selenium, infliximab or other anti-TNFα antibodies or a TSHR inhibitor is recorded in Patent 202180081949.7.

In some preferred embodiments, the TAO manifests as one or more of the following symptoms: eyelid syndrome, exophthalmos, diplopia, ocular motility disorder, chemosis, optic neuropathy, keratopathy, conjunctival lesion and keratopathy and systemic symptoms of patients accompanied by hyperthyroidism.

In some preferred embodiments, the diplopia includes constant diplopia, non-constant diplopia and intermittent diplopia.
(1) Eyelid syndrome: Eyelid syndrome is an important sign of a symptomatic episode of TAO and mainly manifests as the widening of palpebral fissures and the exposure of a portion of scleras called eyelid retraction, and when eyeballs rotate downward, upper eyelids cannot fall along with the eyeballs, which is called upper eyelid lag.
(2) Exophthalmos: TAO mostly manifests as simultaneous exophthalmos of both eyes, but both eyes may suffer from the symptomatic episode sequentially; in an early stage, TAO mostly manifests as axial exophthalmos, and in a later stage, eyeballs are bulging and are fixed at a certain eye position due to fibrosis and contracture of extraocular muscles so that aesthetics is affected; the exophthalmos symptom of patients accompanied by hyperthyroidism tends to develop more quickly; after hyperthyroidism is controlled, some patients experience more apparent exophthalmos.
(3) Diplopia and ocular motility disorder: The extraocular muscles are inevitably affected by TAO, resulting in edema and inflammatory infiltration in an early stage and fibrosis in a later stage; multiple extraocular muscles are involved and may sequentially suffer from a symptomatic episode with different degrees in patients; diplopia may manifest as diplopia at multiple eye positions, and in a late stage, diplopia is constant; frequencies of the involvement of the extraocular muscles are inferior recti, medial recti, superior recti and lateral recti in sequence; and rectus fibrosis can cause a restrictive extraocular muscle lesion.
(4) Chemosis: Bulbar conjunctival hyperemia and tortuosity and dilation of blood vessels mostly develop at insertions of horizontal muscles; a swelling caused by severe chemosis can protrude to an outside of a palpebral fissure and is semi-transparent or cloudy, resulting in the incomplete closure of the palpebral fissure and a secondary corneal lesion.
(5) Optic neuropathy: According to a foreign report, optic neuropathy develops in 5% to 10% of patients with Graves' ophthalmopathy, and irreversible loss of visual function may develop in 30% of the patients and is common in the elderly, males and smokers; the reason is that hypertrophic orbital tissues and muscles due to edema cause an increase in an intra-orbital pressure and optic nerves are compressed at orbital apexes, resulting in a decreased vision and a visual field defect (especially at a lower portion); some patients may experience an elevated intraocular pressure.
(6) Keratopathy: Exposure keratopathy develops due to incomplete closure of palpebral fissure caused by changes in eyelids and severe exophthalmos; in an early stage, punctate epithelial detachment develops below corneas, and in a severe case, corneal ulcers or even perforation may develop, resulting in severe damage to the visual function.
(7) Conjunctival lesion and keratopathy: An intra-orbital soft tissue edema and an increased intra-orbital pressure can cause conjunctival congestion and edema, and in a severe case, a conjunctiva protrudes to an outside of a palpebral fissure.
(8) Patients accompanied by hyperthyroidism still have systemic symptoms such as impatience, an increased basal metabolic rate, an accelerated pulse, weight loss, an increased appetite and hand tremor.

As used herein, "thyroid-associated ophthalmopathy" (TAO), "thyroid eye disease" (TED), "Graves' ophthalmopathy" or "Graves' orbitopathy" (GO) refers to the same disorder or condition and may be used interchangeably. All refer to inflammatory orbital pathologies associated with some autoimmune thyroid disorders, most commonly associated with "Graves' disease" (GD), but sometimes associated with other diseases (for example, Hashimoto's thyroiditis).

The terms "proptosis" and "exophthalmos" (also referred to as exophthalmos/exophthalmia/exorbitism) refer to the forward protrusion, displacement, bulging or prominence of organs. As used herein, the terms refer to the forward protrusion, displacement, bulging or prominence in the case where the eyes move forward away from the orbits. Some skilled in the art think that proptosis and exophthalmos have the same meaning and may be generally used interchangeably, while others think that there is a subtle difference in their meanings. Some people use exophthalmos to refer to severe proptosis or refer to endocrine-related proptosis. Others use the term "exophthalmos" when describing proptosis related to, for example, eyes of subjects with TAO (TED or GO).

As used herein, the terms "proptosis" and "exophthalmos" may be used interchangeably and refer to the forward protrusion, displacement, bulging or prominence in the case where the eyes move forward away from the orbits. Since the orbits only have the rigid bony structures with an anterior opening for expansion, any increase in the orbital soft tissue contents that develops at the lateral or posterior causes the eyeballs to displace forward. Proptosis or exophthalmos may be the result of several disease processes, and the disease processes include infection, inflammation, tumors, wounds, cancer metastasis, endocrinopathy, vascular diseases and extraorbital lesions. TAO (TED or GO) is currently recognized as the most common cause of proptosis in adults. Exophthalmos may be bilateral, which is, for example, common in TAO (TED or GO), or exophthalmos may also be unilateral (for example, common in orbital tumors).

In some preferred embodiments, the thioantisense oligonucleotide targeting the IGF-1R gene or the composition thereof is formulated as a lyophilized agent or an injection.

In some preferred embodiments, the thioantisense oligonucleotide targeting the IGF-1R gene or the composition thereof is administered in combination with one or more TAO treatments.

In some preferred embodiments, the one or more TAO treatments are radiotherapy or/and surgical treatments.

In some preferred embodiments, a dose of the thioantisense oligonucleotide targeting the IGF-1R gene includes 2.5-10 mg/kg.

The present application discovered that an antisense oligonucleotide S-ASODN-1 with a particular sequence targeting an IGF-1R can effectively reduce concentrations of the IGF-1R and an anti-TPO-Ab, thereby improving a level of TSH for treating TAO. In essence, among existing drugs for treating TAO that have been approved or have entered a clinical stage at present, there is no antisense oligonucleotide. Moreover, not all antisense oligonucleotides targeting IGF-1R and having any sequence can treat TAO. For example, other antisense oligonucleotide sequences S-ASODN-2, S-ASODN-3, S-ASODN-4 and S-ASODN-5 in the present application cannot effectively regulate TPO-Ab and TSH levels and cannot be used for treating TAO.

### BRIEF DESCRIPTION OF DRAWINGS

To more clearly illustrate solutions in examples of the present application or the related art, the drawings to be used in the description of the examples or the related art will be briefly described below.
FIG. 1 illustrates curves of variations of weights of rats during administration.
FIG. 2 illustrates variations of TPO-Ab concentrations (IU/mL, Mean±SEM) after S-ASODN-1 acts on rat TAO models.
FIG. 3 illustrates variations of TSH concentrations (mU/L, Mean±SEM) after S-ASODN-1 acts on rat TAO models.
FIG. 4 illustrates variations of IGF-1R concentrations (ng/mL, Mean±SEM) after S-ASODN-1 acts on rat TAO models.
FIGS. 5-1 to 5-10 are pathological images of extraocular muscle lesions after a control group, a model group, an S-ASODN-1 low-dose group, an S-ASODN-1 medium-dose group, an S-ASODN-1 high-dose group, an S-ASODN-2 high-dose group, an S-ASODN-3 high-dose group, an S-ASODN-4 high-dose group, an S-ASODN-5 high-dose group and a positive drug group act on rat TAO models (magnification is 200 times).
FIG. 6-1 illustrates curves of variations of weights of rats during administration in a comparative experiment.
FIG. 6-2 illustrates variations of TPO-Ab concentrations (IU/mL, Mean±SEM) after S-ASODN-1 acts on rat TAO models in a comparative experiment.
FIG. 6-3 illustrates variations of TSH concentrations (mU/L, Mean±SEM) after S-ASODN-1 acts on rat TAO models in a comparative experiment.
FIG. 6-4 illustrates variations of IGF-1R concentrations (ng/mL, Mean±SEM) after S-ASODN-1 acts on rat TAO models in a comparative experiment.

### DETAILED DESCRIPTION

The present application discloses a use of an antisense oligonucleotide in preparation of a drug for treating a disease caused by an abnormal thyroid. Those skilled in the art may use the content herein for reference and appropriately improve the process parameter to implement the use. It is to be particularly noted that all similar substitutions and modifications are apparent to those skilled in the art and are considered to be included in the present application. The method and use of the present application have been described through preferred embodiments, and relevant personnel can apparently make modifications or appropriate changes and combinations to the method and use described herein without departing from the content, spirit and scope of the present application to implement and apply the technology of the present application.

In the use of the antisense oligonucleotide in the preparation of the drug for treating the disease caused by the abnormal thyroid provided in the present application, the raw materials and reagents used may all be purchased from the market.

The present application is further described below in conjunction with examples.

### Example 1: Synthesis of full thioantisense oligonucleotides

### 1. S-ASODN-1 (sequence: 5'-TCCTCCGGAGCCAGACTTCA-3' (SEQ ID NO: 1))

Synthesis was performed through a solid-phase synthesis method, and an instrument used was an OligoPilot 400 synthesizer from General Electric (GE) Company in the United States. Synthesis steps are described below.

### (1) Deprotection

A toluene solution containing dichloroacetic acid was used as a deprotection reagent to remove a 5'-DMT protecting group of a starting nucleoside dA(bz) on a vector and release a 5'-hydroxyl group.

### (2) Coupling

Acetonitrile was used as a solvent, and 5-ethylthiotetrazole was used as an activator to activate a dC(bz) phosphoramidite monomer to form an active intermediate. Then, the active intermediate underwent a condensation reaction with the 5'-hydroxyl group of the nucleoside dA(bz) for coupling.

### (3) Thio

Xanthane hydride was a thio reagent that oxidized phosphite into stable thiophosphate.

### (4) Hydroxyl group protection

Acetic anhydride was used as a protection reagent to protect the 5'-hydroxyl group of the nucleoside that had not undergone the coupling reaction.

According to the nucleotide sequence of S-ASODN-1, the above steps (1) to (4) were repeated to sequentially couple corresponding nucleosides until the coupling of the S-ASODN sequence was complete.

### (5) Deprotection

Dichloroacetic acid was a deprotection reagent to remove a DMT protecting group of the last nucleoside dT to obtain S-ASODN-1 ligated to the vector.

### (6) Aminolysis

A concentrated ammonia solution was added for an aminolysis reaction, an ester bond between the vector and the nucleotide was hydrolyzed, and protecting groups on phosphate, adenine, guanine and cytosine were removed. Filtration was performed, rinsing was performed with an aqueous ethanol solution, and a filtrate was collected.

### (7) Purification

The filtrate was subjected to reverse phase column chromatography and lyophilized to obtain a product with a purity of 93.2%.

Synthesis methods of S-ASODN-2 with a sequence of 5'-TTCATTCCTTTTATTTGGGA-3' (SEQ ID NO: 2), S-ASODN-3 with a sequence of 5'-GGACCCTCCTCCGGAGCC-3' (SEQ ID NO: 3), S-ASODN-4 with a sequence of 5'-GAGAAACAGGAGCCCCCACA-3' (SEQ ID NO: 4) and S-ASODN-5 with a sequence of 5'-GCGCGGCTGGAAAGCGCGTT-3' (SEQ ID NO: 5) were the same as above, and purities were 91.1%, 93.5%, 92.4% and 92.8%, respectively.

### Example 2: Pharmacodynamic experiment of rat TAO models

### 1. Experimental materials

Test samples: the antisense oligonucleotides S-ASODN-1 (sequence: 5'-TCCTCCGGAGCCAGACTTCA-3' (SEQ ID NO:1)), S-ASODN-2 (sequence: 5'-TTCATTCCTTTTATTTGGGA-3' (SEQ ID NO: 2)), S-ASODN-3 (sequence: 5'-GGACCCTCCTCCGGAGCC-3' (SEQ ID NO: 3)), S-ASODN-4 (sequence: 5'-GAGAAACAGGAGCCCCCACA-3' (SEQ ID NO: 4)) and S-ASODN-5 (sequence: 5'-GCGCGGCTGGAAAGCGCGTT-3' (SEQ ID NO: 5)).

Positive drug: Teprotumumab (500 mg/bottle, Horizon Therapeutics USA, Inc.).

Drug solvent: sodium chloride injection.

Reagents used: thyroglobulin (Article No.: T885815-100 mg; Shanghai Macklin Biochemical Technology Co., Ltd.), isoflurane (Lot No.: 20221201, 100 mL, Jiangsu Hengfeng Strong Biological Technology Co., Ltd.), Freund's Complete Adjuvant (5 mL/bottle, Chondrex), TSH Elisa kit (96 T, Wuhan Huamei Biotech Co., Ltd.), TPO-Ab Elisa kit (96 T, Wuhan Huamei Biotech Co., Ltd.) and IGF-1R Elisa kit (48 T, Wuhan Huamei Biotech Co., Ltd.).

Laboratory animals: Sixty 7-week-old female specific-pathogen-free (SPF) Wistar rats weighing 164.7-179.9 g, purchased from Beijing Vital River Laboratory Animal Co., Ltd.; the test animals were housed in sterile individually ventilated cages (IVCs), with six animals per cage; padding was sterilized corn cob padding; sterilized feed specially prepared for the rats was fed, and purified water was drunk freely; the animal laboratory was maintained at a temperature of around 25 °C, kept at a relative humidity of 40% to 70% and exposed to light for 12 h per day.

### 2. Experimental method

### 2.1 Animal grouping and model establishment

The sixty 7-week-old female SPF Wistar rats weighing 164.7-179.9 g were randomly divided into a control group (0.9% NaCl, 50 µL/eye, qw×4), a model group (0.9% NaCl, 50 µL/eye, qw×4), an S-ASODN-1 low-dose group (2.5 mg/kg, 50 µL/eye, qw×4), an S-ASODN-1 medium-dose group (5 mg/kg, 50 µL/eye, qw×4), an S-ASODN-1 high-dose group (10 mg/kg, 50 µL/eye, qw×4), an S-ASODN-2 high-dose group (10 mg/kg, 50 µL/eye, qw×4), an S-ASODN-3 high-dose group (10 mg/kg, 50 µL/eye, qw×4), an S-ASODN-4 high-dose group (10 mg/kg, 50 µL/eye, qw×4), an S-ASODN-5 high-dose group (10 mg/kg, 50 µL/eye, qw×4) and a positive drug group (Teprotumumab, 10 mg/kg, tail vein injection) according to weights, 6 rats/group. Animal grouping information is shown in Table 4.

**Table 4 Animal grouping information table**

| Group | | Test Article/Control Substance | Administration Dosage | | Administration Frequency | **Animal** No. |
|---|---|---|---|---|---|---|
| | | | Adminis tration Dosage (mg/kg) | Admini stration Volume (µL) | | |
| 1 | control group | normal saline | - | - | qw×4 | 1F001~1F 006 |
| 2 | model group | normal saline | - | 50 | qw×4 | 2F001~2F 006 |
| 3 | S-ASODN-1 low-dose group | S-ASODN-1 | 2.5 | 50 | qw×4 | 3F001~3F 006 |
| 4 | S-ASODN-1 medium-dose group | S-ASODN-1 | 5 | 50 | qw×4 | 4F001~4F 006 |
| 5 | S-ASODN-1 high-dose group | S-ASODN-1 | 10 | 50 | qw×4 | 5F001~5F 006 |
| 6 | S-ASODN-2 high-dose group | S-ASODN-2 | 10 | 50 | qw×4 | 6F001~6F 006 |
| 7 | S-ASODN-3 high-dose group | S-ASODN-3 | 10 | 50 | qw×4 | 7F001~7F 006 |
| 8 | S-ASODN-4 high-dose group | S-ASODN-4 | 10 | 50 | qw×4 | 8F001~8F 006 |
| 9 | S-ASODN-5 high-dose group | S-ASODN-5 | 10 | 50 | qw×4 | 9F001~9F 006 |
| 10 | positive drug group | teprotumumab | 10 | 50 | qw×4 | 10F001~1 0F006 |

### Notes:

[1] The first digit of the animal No. represents the group, the second alphabetic letter represents the gender (F is female, and M is male), and the third, the fourth and the fifth digits represent the individual animal No.
[2] The "qw×4" represents administration once a week for a total of four administrations.

### 2.2 Experimental treatment method

Except for the control group, each group was fed with thyroglobulin and a Freund's Incomplete/Complete Adjuvant emulsion through a 0.6% aqueous NaI solution according to an intraperitoneal injection method to establish a Wistar rat TAO model. Oculus Uterque (OU) posterior injection administration or intravenous injection administration began on the 27th day of modeling that was denoted as D1 (or M27). Administration was performed once a week for four consecutive weeks, and the days were denoted as D2 (M28), D3 (M29), D4 (M30), ..., respectively. The day before D1 of administration was denoted as D0.

### 2.3 Evaluation indicators

Clinical observation, weights, concentrations of TPO-Abs and TSH in serums on D0 and D23, concentrations of IGF-1Rs in intact Oculus Dexter (OD) tissues and pathological examination of OU extraocular muscle tissues.

Weight measurement: Weight measurement was performed on the day of animal reception, the day of quarantine completion and the day of grouping and was performed once a week after grouping; an animal was weighed when found dead or dying.

Protein detection: Serums were taken from all the animals in the groups on D0 and D23, and the TPO-Abs and the TSH were measured through ELISA; OD eyeballs were taken from each group of animals on D23, and the IGF-1Rs were measured through ELISA.

Pathological examination: Extraocular muscle tissues were taken from all surviving animals in the groups (all animals) on M49 (D23), and lesions were observed with naked eyes; after the tissues were fixed for at least 48 h, the extraocular muscle tissues and thyroid tissues of all planned and unplanned dissected animals were sampled, dehydrated, embedded and sliced and were subjected to hematoxylin-eosin stain and image reading.

### 2.4 Data processing

Descriptive analysis was used for data such as a general condition and histopathology. For quantitative indicators such as a weight, mean±standard error of the mean (mean±SEM) was calculated according to a group. A comparison between two groups was checked using t, and P < 0.05 indicated that a statistical difference was significant. For a comparison between multiple groups, data of each indicator were analyzed according to the following procedure: equal variance was checked through a Levene's test. If the variance was equal (P > 0.05), a statistical analysis was performed through an analysis of variance (ANOVA).If the ANOVA had statistical significance (P ≤ 0.05), a comprehensive comparison was performed through a least significant difference (LSD) method. If the variance was not equal (P ≤ 0.05), the statistical analysis was performed through a Dunnett's T3 test.

### The above statistical operations were performed using SPSS 25.0.

### 3. Experimental results

### 3.1 Observation of weights and general states

During the experiment, Animal No. 2F003 in the model group experienced bloating on M27 (D1) and died on M29 (D3), Animal No. 4F005 in the S-ASODN-1 medium-dose group died on M46 (D20), and no abnormal signs were observed in other groups of animals during the experiment.

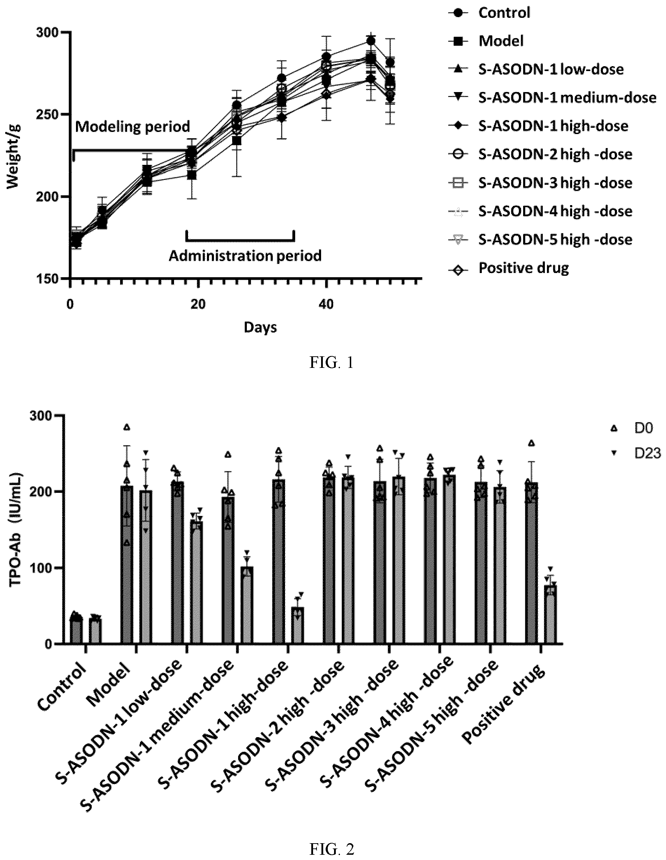

On D23, average fasting weights of Wistar rats in the control group, the model group, the S-ASODN-1 low-dose group, the S-ASODN-1 medium-dose group, the S-ASODN-1 high-dose group, the S-ASODN-2 high-dose group, the S-ASODN-3 high-dose group, the S-ASODN-4 high-dose group, the S-ASODN-5 high-dose group and the positive drug group are 281.78±5.85 g, 270.48±6.05 g, 273.35±4.73 g, 258.58±3.28 g, 259.38±6.24 g, 267.52±2.07 g, 267.47±1.76 g, 271.97±2.74 g, 271.85±3.05 g, 262.40±2.55 g, respectively. During the experiment, compared with the normal control group, weight gains of Wistar rats in the model group are slow, and weights on M19, D0 and D7 are decreased significantly (P < 0.05); compared with the model group, weight growth trends of Wistar rats in the S-ASODN-1 low-dose group, the S-ASODN-1 medium-dose group, the S-ASODN-1 high-dose group, the S-ASODN-2 high-dose group, the S-ASODN-3 high-dose group, the S-ASODN-4 high-dose group, the S-ASODN-5 high-dose group and the positive drug group during administration are consistent, and no statistical differences exist between the groups (P > 0.05).Reference may be made to FIG. 1.

### 3.2 TPO-Ab protein detection

A high level of TPO-Ab indicates that a human immune system has launched an attack on thyroid gland. This situation is generally associated with autoimmune thyroid diseases such as TAO.

The concentrations of the TPO-Abs in the serums of the Wistar rats in the control group, the model group, the S-ASODN-1 low-dose group, the S-ASODN-1 medium-dose group, the S-ASODN-1 high-dose group, the S-ASODN-2 high-dose group, the S-ASODN-3 high-dose group, the S-ASODN-4 high-dose group, the S-ASODN-5 high-dose group and the positive drug group on D0 before administration and D23 after administration are shown in the following table and FIG. 2.

**Table 5**

| Group | TPO-Ab Concentration (IU/mL) | | Concentration Variation (D 1 to D23) (IU/mL) | Comparison with Model Group |
|---|---|---|---|---|
| | D0 | D23 | | |
| control group | 35.81±1.03 | 33.09±1.01 | 2.72 | / |
| model group | 207.68±21.47 | 201.69±18.06 | 5.99 | 1 |
| S-ASODN-1 low-dose group | 231.30±5.12 | 161.48±4.20 | 69.82 | 11.65609 |
| S-ASODN-1 medium-dose group | 193.11±13.58 | 102.03±5.68 | 91.08 | 15.20534 |
| S-ASODN-1 high-dose group | 216.12±12.25 | 48.57±4.56 | 167.55 | 27.97162 |
| S-ASODN-2 high-dose group | 218.66±5.56 | 218.60±5.98 | 0.06 | 0.010017 |
| S-ASODN-3 high-dose group | 213.86±11.74 | 219.76±9.75 | -5.90 | -0.98497 |
| S-ASODN-4 high-dose group | 217.90±8.07 | 222.14±3.32 | -4.24 | -0.70785 |
| S-ASODN-5 high-dose group | 212.91±8.56 | 206.16±8.72 | 6.75 | 1.126878 |
| positive drug group | 212.28±11.02 | 77.50±5.23 | 134.78 | 22.50083 |

During the experiment, compared with the control group, the concentrations of the TPO-Abs in the serums of the Wistar rats in other groups on D0 before administration are increased significantly (P < 0.001), proving that the mouse TAO model is successfully established in each group.

Compared with D0 before administration in each group, the concentrations of the TPO-Abs in the serums of the Wistar rats in the S-ASODN-1 low-dose group, the S-ASODN-1 medium-dose group, the S-ASODN-1 high-dose group and the positive drug group are decreased by about 70-170 IU/mL after the last administration on D23, and the decrease in the concentration of the TAO-Ab is about 10 to 30 times that of the model group. The concentration is reduced significantly (P < 0.001), exhibiting a good therapeutic effect. The concentration of the TPO-Ab of the S-ASODN-1 high-dose group is only 48.57 IU/mL after the last administration on D23, and the decrease in the concentration of the TPO-Ab is about 27.97 times that of the model group, exhibiting the most excellent therapeutic effect.

Compared with D0 before administration in each group, no statistical differences (P > 0.05) exist in the S-ASODN-2 high-dose group, the S-ASODN-3 high-dose group, the S-ASODN-4 high-dose group, the S-ASODN-5 high-dose group and the model group after the last administration on D23. No significant variations exist in the concentrations of the TPO-Abs of the S-ASODN-2 high-dose group, the S-ASODN-3 high-dose group, the S-ASODN-4 high-dose group and the S-ASODN-5 high-dose group, resulting in a relatively poor therapeutic effect or no therapeutic effect.

In the positive drug group, the decrease in the concentration of the TPO-Ab is about 22.50 times that of the model group. As can be seen from the above data, an order of therapeutic effects of each group is S-ASODN-1 high-dose group > positive drug group > S-ASODN-1 medium-dose group > S-ASODN-1 low-dose group > S-ASODN-2 high-dose group, S-ASODN-3 high-dose group, S-ASODN-4 high-dose group and S-ASODN-5 high-dose group.

### 3.3 TSH protein detection

Under normal circumstances, thyroid-stimulating hormone (TSH) secreted by pituitary gland, also known as thyrotropic hormone, regulates the secretion of thyroxine, while thyrotropin-releasing hormone (TRH) in a hypothalamus regulates the secretion of TSH. A general negative feedback inhibition exists between the thyroid hormone and the TSH, that is, when a level of the thyroid hormone increases, the secretion of the TSH is inhibited, while when the level of the thyroid hormone is low, the pituitary gland is stimulated, and the secretion of the TSH is increased. The decrease in the level of the thyroxine (T2 or T6) in a serum causes an increase in a level of follicle-stimulating hormone (FSH) in the serum and an enhanced response of the TSH to the stimulation of the TRH. Thyroid dysfunction changes the above regulations and responses, and a state of thyroid function is closely associated with TAO. Therefore, understanding the state of thyroid function is of great significance for the diagnosis of TAO.

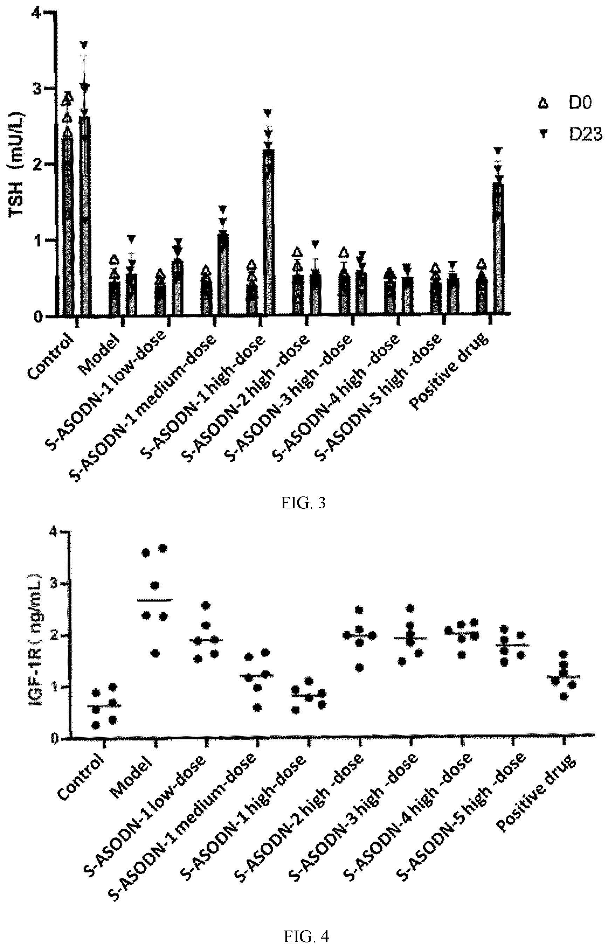

The concentrations of the TSH in the serums of the Wistar rats in the control group, the model group, the S-ASODN-1 low-dose group, the S-ASODN-1 medium-dose group, the S-ASODN-1 high-dose group, the S-ASODN-2 high-dose group, the S-ASODN-3 high-dose group, the S-ASODN-4 high-dose group, the S-ASODN-5 high-dose group and the positive drug group on D0 before administration and after the last administration on D23 are shown in the following table and FIG. 3.

**Table 6**

| Group | TSH Concentration (IU/mL) | | Concentration Variation (D23 to D1) (IU/mL) | Comparison with Model Group |
|---|---|---|---|---|
| | D0 | D23 | | |
| control group | 2.35±0.24 | 2.64±0.32 | 0.29 | / |
| model group | 0.45±0.07 | 0.56±0.11 | 0.11 | 1 |
| S-ASODN-1 low-dose group | 0.40±0.04 | 0.72±0.08 | 0.32 | 2.909091 |
| S-ASODN-1 medium-dose group | 0.43±0.05 | 1.07±0.08 | 0.64 | 5.818182 |
| S-ASODN-1 high-dose group | 0.48±0.08 | 2.18±0.12 | 1.70 | 15.45455 |
| S-ASODN-2 high-dose group | 0.52±0.08 | 0.53±0.08 | 0.01 | 0.090909 |
| S-ASODN-3 high-dose group | 0.50±0.07 | 0.56±0.07 | 0.06 | 0.545455 |
| S-ASODN-4 high-dose group | 0.44±0.05 | 0.49±0.04 | 0.05 | 0.454545 |
| S-ASODN-5 high-dose group | 0.42±0.06 | 0.47±0.04 | 0.05 | 0.454545 |
| positive drug group | 0.45±0.06 | 1.71±0.12 | 1.26 | 11.45455 |

During the experiment, compared with the control group, the concentrations of the TSH in the serums of the Wistar rats in other groups on D0 before administration are decreased significantly (P < 0.001), proving that the mouse TAO model is successfully established in each group.

Compared with D0 before administration in each group, after the last administration on D23, the concentration of the TSH in the serum of the Wistar rats in the S-ASODN-1 low-dose group is increased significantly (P < 0.05), and the concentrations of the TSH in the serums of the Wistar rats in the S-ASODN-1 medium-dose group, the S-ASODN-1 high-dose group and the positive drug group are increased significantly (P < 0.001).For the above three groups, the concentration of the TSH is increased to 0.64-1.70 IU/mL, and the increase is about 3 to 15 times that of the model group, exhibiting a good therapeutic effect. The concentration of the TSH of the S-ASODN-1 high-dose group is increased to 2.18 IU/mL after the last administration on D23, and the decrease is about 15.45 times that of the model group, exhibiting the most excellent therapeutic effect.

No statistical differences (P > 0.05) exist in the S-ASODN-2 high-dose group, the S-ASODN-3 high-dose group, the S-ASODN-4 high-dose group, the S-ASODN-5 high-dose group and the model group. No significant variations exist in the concentrations of the TSH of the S-ASODN-2 high-dose group, the S-ASODN-3 high-dose group, the S-ASODN-4 high-dose group and the S-ASODN-5 high-dose group before and after administration, resulting in a relatively poor therapeutic effect or no therapeutic effect.

In the positive drug group, the increase in the concentration of the TSH is about 11.45 times that of the model group. As can be seen from the above data, an order of therapeutic effects of each group is S-ASODN-1 high-dose group > positive drug group > S-ASODN-1 medium-dose group > S-ASODN-1 low-dose group > S-ASODN-2 high-dose group, S-ASODN-3 high-dose group, S-ASODN-4 high-dose group and S-ASODN-5 high-dose group.

### 3.4 IGF-1R detection

The results of detecting the concentrations of the IGF-1Rs in the intact OD tissues on D23 are shown in the following table and FIG. 4:

**Table 7**

| Group | IGF-1R Concentration (ng/mL) |
|---|---|
| | D23 |
| control group | 0.63±0.12 |
| model group | 2.77±0.32 |
| S-ASODN-1 low-dose group | 1.95±0.15 |
| S-ASODN-1 medium-dose group | 1.19±0.16 |
| S-ASODN-1 high-dose group | 0.80±0.08 |
| S-ASODN-2 high-dose group | 1.94±0.15 |
| S-ASODN-3 high-dose group | 1.92±0.15 |
| S-ASODN-4 high-dose group | 1.97±0.09 |
| S-ASODN-5 high-dose group | 1.75±0.10 |
| positive drug group | 1.16±0.12 |

During the experiment, compared with the control group, the concentrations of the IGF-1Rs in the OD tissues of the Wistar rats in the model group, the S-ASODN-1 low-dose group, the S-ASODN-2 high-dose group, the S-ASODN-3 high-dose group, the S-ASODN-4 high-dose group and the S-ASODN-5 high-dose group are increased significantly (P < 0.001), the concentrations of the IGF-1Rs in the OD tissues of the Wistar rats in the S-ASODN-1 medium-dose group and the positive drug group are increased significantly (P < 0.05), and no statistical difference exists in the concentration of the IGF-1R in the OD tissues of the Wistar rats in the S-ASODN-1 high-dose group (P > 0.05).

Compared with the model group, the concentrations of the IGF-1Rs in the OD tissues of the Wistar rats in the positive drug group and the S-ASODN-1 high-dose group are decreased significantly (P < 0.001), the concentrations of the IGF-1Rs in the OD tissues of the Wistar rats in the S-ASODN-1 low-dose group, the S-ASODN-2 high-dose group, the S-ASODN-3 high-dose group, the S-ASODN-4 high-dose group and the S-ASODN-5 high-dose group are decreased significantly (P < 0.05), and the concentration of the IGF-1R in the OD tissues of the Wistar rats in the S-ASODN-1 medium-dose group is decreased significantly (P < 0.01).

### 3.5 Pathological examination

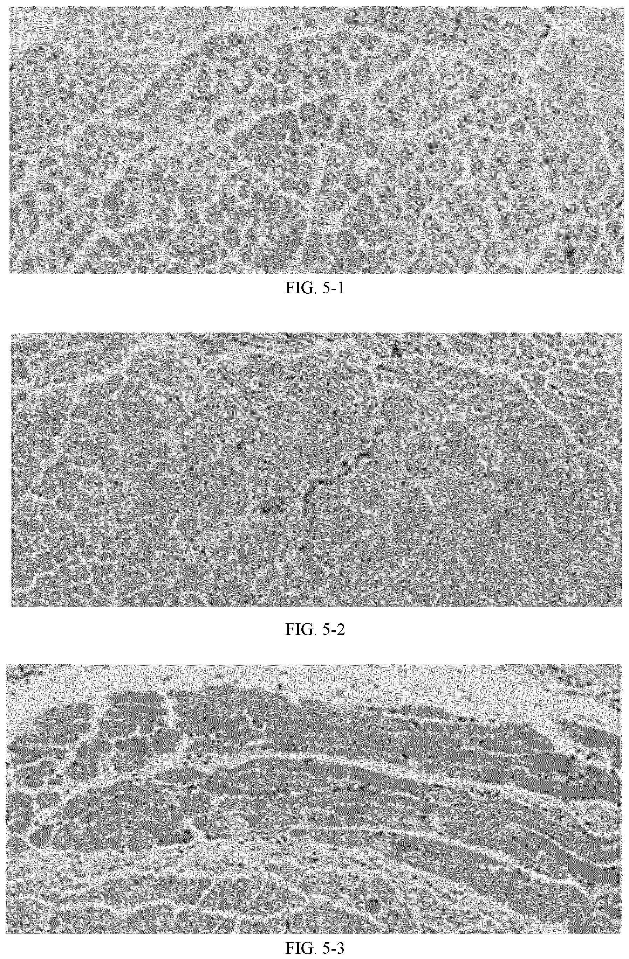

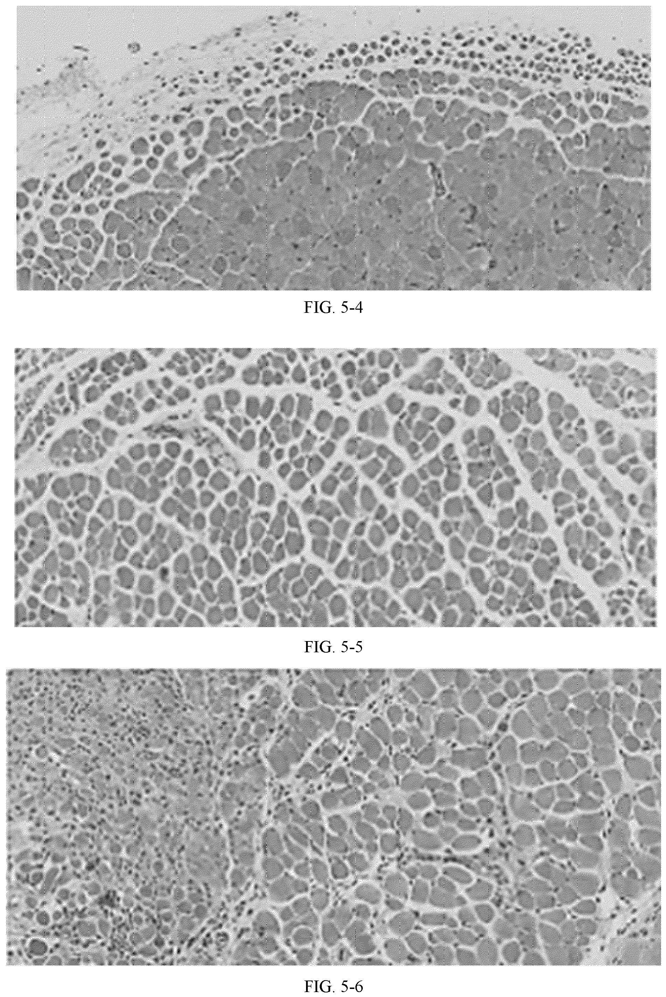

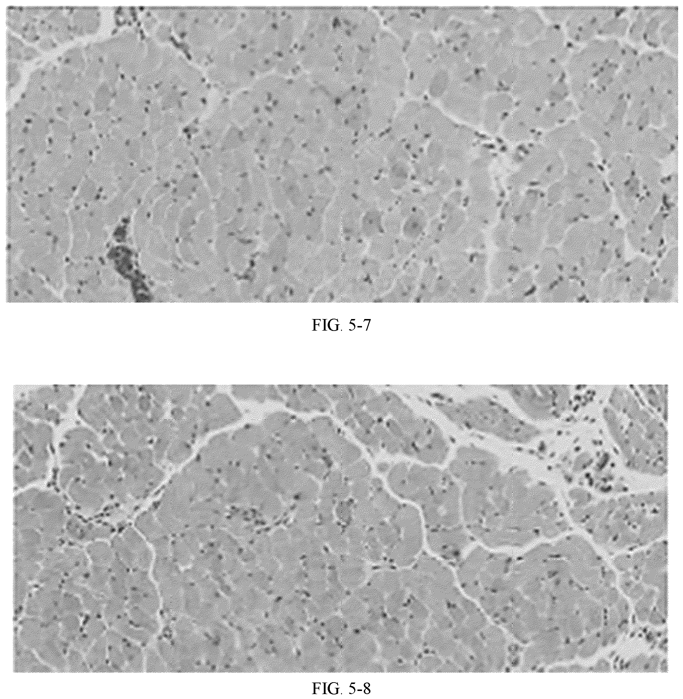

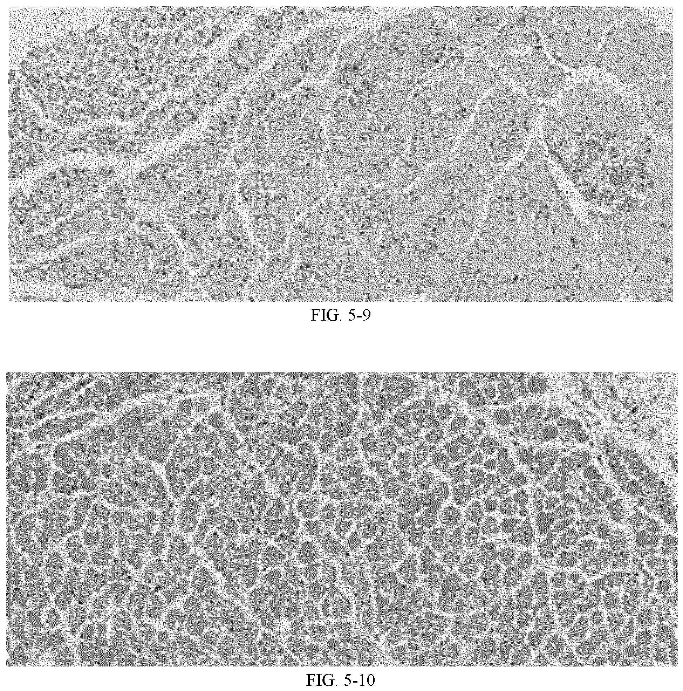

Situations obtained from the pathological images of the extraocular muscle lesions of the rat TAO models (the magnification is 200 times) shown in FIGS. 5-1 to 5-10 are described below.

Control group: No significant abnormalities were observed in extraocular muscles of Oculus Sinister and Oculus Dexter of 6/6 of the animals.

Model group: Mild swelling of extraocular muscle fibers was observed in extraocular muscles of Oculus Sinister of 1/5 of the animals, mild degeneration and necrosis of muscle fibers were observed in extraocular muscles of Oculus Sinister of 4/5 of the animals, and mild degeneration and necrosis of muscle fibers were observed in extraocular muscles of Oculus Dexter of 5/5 of the animals.

S-ASODN-1 low-dose group: Mild swelling of extraocular muscle fibers was observed in extraocular muscles of Oculus Sinister of 3/6 of the animals, mild degeneration and necrosis of muscle fibers were observed in extraocular muscles of Oculus Sinister of 3/6 of the animals, mild swelling of extraocular muscle fibers was observed in extraocular muscles of Oculus Dexter of 4/6 of the animals, and mild degeneration and necrosis of muscle fibers were observed in extraocular muscles of Oculus Dexter of 2/6 of the animals.

S-ASODN-1 medium-dose group: Mild swelling of extraocular muscle fibers was observed in extraocular muscles of Oculus Sinister of 4/5 of the animals, mild degeneration and necrosis of muscle fibers were observed in extraocular muscles of Oculus Sinister of 1/5 of the animals, mild swelling of extraocular muscle fibers was observed in extraocular muscles of Oculus Dexter of 3/5 of the animals, and mild degeneration and necrosis of muscle fibers were observed in extraocular muscles of Oculus Dexter of 2/5 of the animals.

S-ASODN-1 high-dose group: No significant abnormalities were observed in extraocular muscles of Oculus sinister and Oculus Dexter of 6/6 of the animals.

S-ASODN-2 high-dose group: Mild degeneration and necrosis of muscle fibers were observed in extraocular muscles of Oculus Sinister of 6/6 of the animals, mild swelling of extraocular muscle fibers was observed in extraocular muscles of Oculus Dexter of 1/6 of the animals, and mild degeneration and necrosis of muscle fibers were observed in extraocular muscles of Oculus Dexter of 5/6 of the animals.

S-ASODN-3 high-dose group: Mild swelling of extraocular muscle fibers was observed in extraocular muscles of Oculus Sinister of 1/6 of the animals, mild degeneration and necrosis of muscle fibers were observed in extraocular muscles of Oculus Sinister of 5/6 of the animals, mild swelling of extraocular muscle fibers was observed in extraocular muscles of Oculus Dexter of 3/6 of the animals, and mild degeneration and necrosis of muscle fibers were observed in extraocular muscles of Oculus Dexter of 3/6 of the animals.

S-ASODN-4 high-dose group: Mild swelling of extraocular muscle fibers was observed in extraocular muscles of Oculus Sinister of 1/6 of the animals, mild degeneration and necrosis of muscle fibers were observed in extraocular muscles of Oculus Sinister of 5/6 of the animals, and mild degeneration and necrosis of muscle fibers were observed in extraocular muscles of Oculus Dexter of 6/6 of the animals.

S-ASODN-5 high-dose group: Mild swelling of extraocular muscle fibers was observed in extraocular muscles of Oculus Sinister of 1/6 of the animals, mild degeneration and necrosis of muscle fibers were observed in extraocular muscles of Oculus Sinister of 5/6 of the animals, mild swelling of extraocular muscle fibers was observed in extraocular muscles of Oculus Dexter of 2/6 of the animals, and mild degeneration and necrosis of muscle fibers were observed in extraocular muscles of Oculus Dexter of 4/6 of the animals.

Positive drug group: No significant abnormalities were observed in extraocular muscles of Oculus sinister and Oculus Dexter of 6/6 of the animals.

A summary of the number of lesions is shown in Table 8.

**Table 8 Summary of the number of extraocular muscle lesions**

| Group | | Oculus Sinister | | Oculus Dexter | |
|---|---|---|---|---|---|
| | | swelling of muscle fibers | degeneration and necrosis of muscle fibers | swelling of muscle fibers | degeneration and necrosis of muscle fibers |
| 1 | control group (N = 6) | 0 | 0 | 0 | 0 |
| 2 | model group (N = 5) | 1 | 4 | 0 | 5 |
| 3 | S-ASODN-1 low-dose group (N = 6) | 3 | 3 | 4 | 2 |
| 4 | S-ASODN-1 medium-dose group (N = 5) | 4 | 1 | 3 | 2 |
| 5 | S-ASODN-1 high-dose group (N = 6) | 0 | 0 | 0 | 0 |
| 6 | S-ASODN-2 high-dose group (N = 6) | 0 | 6 | 1 | 5 |
| 7 | S-ASODN-3 high-dose group (N = 6) | 1 | 5 | 3 | 3 |
| 8 | S-ASODN-4 high-dose group (N = 6) | 1 | 5 | 0 | 6 |
| 9 | S-ASODN-5 high-dose group (N = 6) | 1 | 5 | 2 | 4 |
| 10 | positive drug group (N = 5) | 0 | 0 | 0 | 0 |

### Conclusion

Under the conditions of this experiment, the orbital tissue lesions in the Wistar rats are induced through repeated intraperitoneal injections of bovine thyroglobulin (Tg), resulting in the significant increase in the level of the TPOAb in the serum of the animals. Consistent disease courses of OU indicate successful rat modeling. Administration is performed in 50 µL/eye through eyeball posterior injection once a week for four consecutive weeks. The S-ASODN-1 high-dose group is superior to the positive drug group in regulating the expression of TPO-Ab proteins in the serum of the rats, the expression of TSH proteins in the serum of the rats and the expression of IGF-1Rs in OD of the rats and also exhibits an effect of reducing the tissue lesions in pathological sections of the extraocular muscles, exhibiting a good therapeutic effect of treating TAO.

### 4. Comparative experiment

### 4.1. Experimental materials

Test sample: thioantisense oligonucleotide S-ASODN-1 (sequence: 5'-TCCTCCGGAGCCAGACTTCA-3' (SEQ ID NO:1)).

Positive drug 2: miR-143 simulant, sense: 5'-CACAGAUAGAAGGGCCUCGU-3' (SEQ ID NO:6), antisense: 5'-CCAGGUGAAGCUACUGCAAG-3' (SEQ ID NO:7).

Drug solvent: sodium chloride injection.

Reagents used: thyroglobulin (Article No.: T885815-100 mg; Shanghai Macklin Biochemical Technology Co., Ltd.), isoflurane (Lot No.: 20221201, 100 mL, Jiangsu Hengfeng Strong Biological Technology Co., Ltd.), Freund's Complete Adjuvant (5 mL/bottle, Chondrex), TSH Elisa kit (96 T, Wuhan Huamei Biotech Co., Ltd.), TPO-Ab Elisa kit (96 T, Wuhan Huamei Biotech Co., Ltd.) and IGF-1R Elisa kit (48 T, Wuhan Huamei Biotech Co., Ltd.).

Laboratory animals: 24 7-week-old female SPF Wistar rats weighing 165.2-175.6 g, purchased from Beijing Vital River Laboratory Animal Co., Ltd.; the test animals were housed in sterile IVCs, with five animals per cage; padding was sterilized corn cob padding; sterilized feed specially prepared for the rats was fed, and purified water was drunk freely; the animal laboratory was maintained at a temperature of around 25 °C, kept at a relative humidity of 40% to 70% and exposed to light for 12 h per day.

### 4.2 Experimental method

### 4.2.1 Animal grouping and model establishment

The 24 7-week-old female SPF Wistar rats weighing 165.2-175.6 g were randomly divided into a control group (0.9% NaCl, 50 µL/eye, qw×4), a model group (0.9% NaCl, 50 µL/eye, qw×4), an S-ASODN-1 high-dose group (10 mg/kg, 50 µL/eye, qw×4) and a positive drug group (miR-143 stimulant, 10 mg/kg, tail vein injection) according to weights, 6 rats/ group. Animal grouping information is shown in the table. Animal grouping information is shown in the table.

**Table 9 Animal grouping information table**

| Group | | Test Article/Control Substance | Administration Dosage | | Administration Frequency | **Animal** No. |
|---|---|---|---|---|---|---|
| | | | Adminis tration Dosage (mg/kg) | Administrati on Volume (µL) | | |
| 1 | control group | normal saline | - | - | qw×4 | 11F001~1 F006 |
| 2 | model group | normal saline | - | 50 | qw×4 | 12F001~2 F006 |
| 3 | S-ASODN -1 high-dose group | S-ASODN-1 | 10 | 50 | qw×4 | 13F001~5 F006 |
| 4 | positive drug 2 group | miR-143 simulant | 10 | 50 | qw×4 | 14F001~1 0F006 |

### Notes:

[1] The first digit of the animal No. represents the group, the second alphabetic letter represents the gender (F is female, and M is male), and the third, the fourth and the fifth digits represent the individual animal No.
[2] The "qw×4" represents administration once a week for a total of four administrations.

### 4.2.2 Experimental treatment solution

Except for the control group, each group was fed with thyroglobulin and a Freund's Incomplete/Complete Adjuvant emulsion through a 0.6% aqueous NaI solution according to an intraperitoneal injection method to establish a Wistar rat TAO model. OU posterior injection administration or intravenous injection administration began on the 27th day of modeling that was denoted as D1. Administration was performed once a week for four consecutive weeks.

### 4.2.3 Evaluation indicators

Clinical observation, weights, concentrations of TPO-Abs and TSH in serums before administration on D1 and on D23, concentrations of IGF-1Rs in intact OD tissues.

Weight measurement: Weight measurement was performed on the day of animal reception, the day of quarantine completion and the day of grouping and was performed once a week after grouping; an animal was weighed when found dead or dying.

Protein detection: Serums were taken from all the animals in the groups on D1 and D23, and the TPO-Abs and the TSH were measured through ELISA; OD eyeballs were taken from each group of animals on D23, and the IGF-1Rs were measured through ELISA.

### 4.2.4 Data processing

Descriptive analysis was used for data such as a general condition and histopathology. For quantitative indicators such as a weight, mean±SEM was calculated according to a group. A comparison between two groups was checked using t, and P < 0.05 indicated that a statistical difference was significant. For a comparison between multiple groups, data of each indicator were analyzed according to the following procedure: equal variance was checked through a Levene's test. If the variance was equal (P > 0.05), a statistical analysis was performed through an ANOVA. If the ANOVA had statistical significance (P ≤ 0.05), a comprehensive comparison was performed through an LSD method. If the variance was not equal (P ≤ 0.05), the statistical analysis was performed through a Dunnett's T3 test.

The above statistical operations were performed using SPSS 25.0.

### 4.3 Experimental results

### 4.3.1 Observation of weights and general states

During the experiment, no abnormal signs were observed in any group of animals.

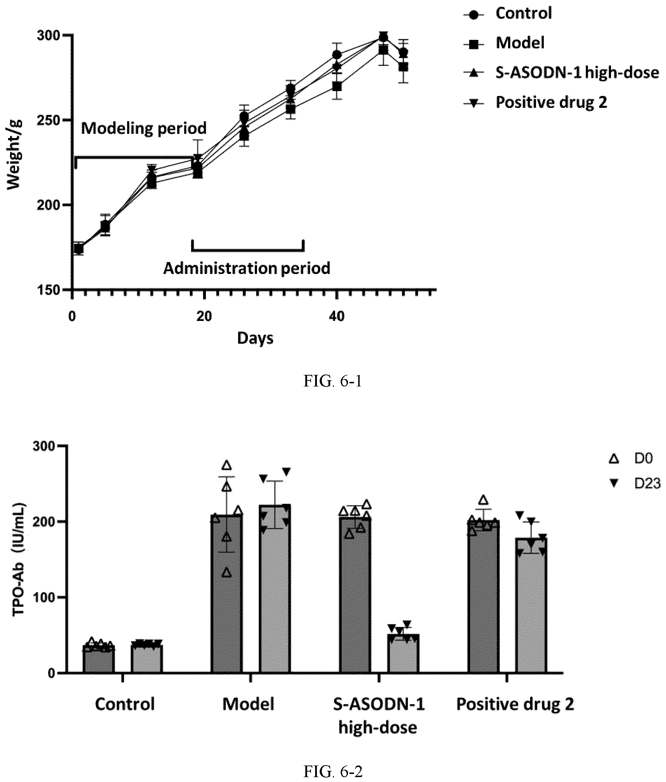

On D23, average fasting weights of Wistar rats in the control group, the model group, the S-ASODN-1 high-dose group and the positive drug 2 group are 290.12±2.98 g, 281.40±3.87 g, 288.87±3.51 g and 289.35±2.41 g, respectively. During the experiment, compared with the normal control group, weight gains of Wistar rats in the model group are slow, and weights on M19, D0 and D7 are decreased significantly (P < 0.01); compared with the model group, weight growth trends of Wistar rats in the S-ASODN-1 high-dose group and the positive drug 2 group during administration are consistent, and no statistical differences exist between the groups (P > 0.05).Reference may be made to FIG. 6-1.

### 4.3.2 TPO-Ab protein detection

A high level of TPO-Ab indicates that a human immune system has launched an attack on thyroid gland. This situation is generally associated with autoimmune thyroid diseases such as TAO.

The concentrations of the TPO-Abs in the serums of the Wistar rats in the control group, the model group, the S-ASODN-1 high-dose group and the positive drug 2 group before administration on D1 and on D23 after administration are shown in the following table and FIG. 6-2.

**Table 10**

| Group | TPO-Ab Concentration (IU/mL) | | Concentration Variation (D1 to D23) (IU/mL) | Comparison with Model Group |
|---|---|---|---|---|
| | D1 | D23 | | |
| control group | 36.93±1.20 | 37.22±0.61 | -0.29 | / |
| model group | 209.34±20.31 | 222.27±12.82 | -12.93 | 1 |
| S-ASODN-1 high-dose group | 206.08±6.09 | 51.80±3.42 | 154.28 | 11.9319 |
| positive drug 2 group | 202.12±5.79 | 178.87±8.48 | 23.25 | 1.7981 |

During the experiment, compared with the control group, the concentrations of the TPO-Abs in the serums of the Wistar rats in other groups before administration on D1 are increased significantly (P < 0.001), proving that the mouse TAO model is successfully established in each group.

Compared with the case before administration on D1 in each group, the concentrations of the TPO-Abs in the serums of the Wistar rats in the S-ASODN-1 high-dose group and the positive drug group are decreased by 23-154 IU/mL after the last administration on D23, and the decrease in the concentration of the TAO-Ab is about 1 to 10 times that of the model group. The concentration is reduced significantly (P < 0.001 or P < 0.05), exhibiting a good therapeutic effect. The decrease in the concentration of the TPO-Ab of the positive drug 2 group is 1.80 times that of the model group. The concentration of the TPO-Ab of the S-ASODN-1 high-dose group is only 51.90 IU/mL after the last administration on D23, and the decrease in the concentration of the TPO-Ab is 11.93 times that of the model group and 6.66 times that of the positive drug 2 group, exhibiting the most excellent therapeutic effect.

As can be seen from the above data, an order of therapeutic effects of each group is S-ASODN-1 high-dose group > positive drug 2 group.

### 4.3.3 TSH protein detection

Under normal circumstances, TSH secreted by pituitary gland, also known as thyrotropic hormone, regulates the secretion of thyroxine, while TRH in a hypothalamus regulates the secretion of TSH.A general negative feedback inhibition exists between the thyroid hormone and the TSH, that is, when a level of the thyroid hormone increases, the secretion of the TSH is inhibited, while when the level of the thyroid hormone is low, the pituitary gland is stimulated, and the secretion of the TSH is increased. The decrease in the level of the thyroxine (T2 or T6) in a serum causes an increase in a level of FSH in the serum and an enhanced response of the TSH to the stimulation of the TRH. Thyroid dysfunction changes the above regulations and responses, and a state of thyroid function is closely associated with TAO. Therefore, understanding the state of thyroid function is of great significance for the diagnosis of TAO.

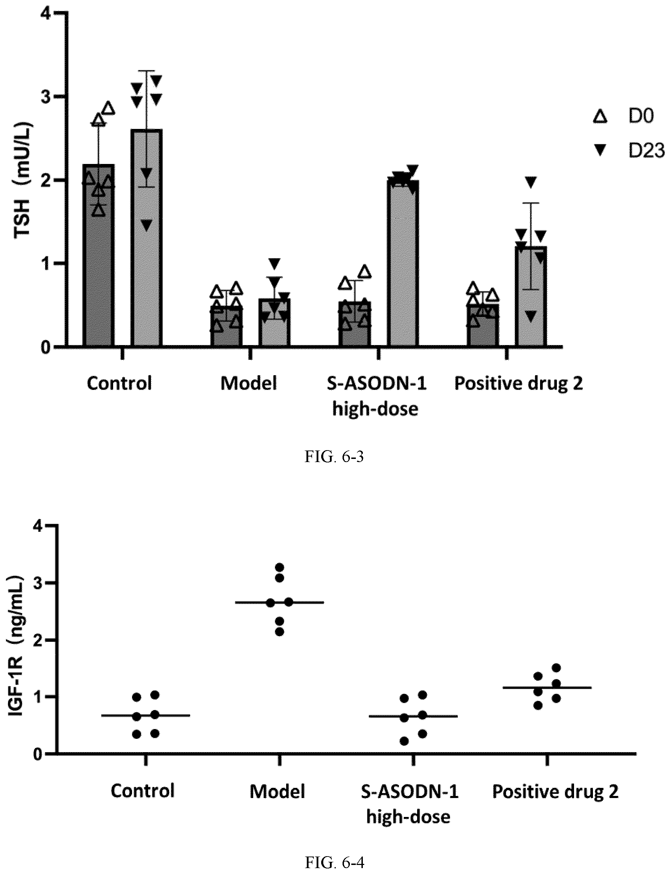

The concentrations of the TSH in the serums of the Wistar rats in the control group, the model group, the S-ASODN-1 high-dose group and the positive drug group before administration on D1 and after the last administration on D23 are shown in the following table and FIG. 6-3.

**Table 11**

| Group | TSH Concentration (IU/mL) | | Concentration Variation (D23 to D1) (IU/mL) | Comparison with Model Group |
|---|---|---|---|---|
| | D1 | D23 | | |
| control group | 2.19±0.20 | 2.61±0.28 | 0.42 | / |
| model group | 0.49±0.07 | 0.58±0.10 | 0.09 | 1 |
| S-ASODN-1 high-dose group | 0.55±0.10 | 2.00±0.03 | 1.45 | 16.1111 |
| positive drug 2 group | 0.52±0.06 | 1.21±0.21 | 0.69 | 7.6667 |

During the experiment, compared with the control group, the concentrations of the TSH in the serums of the Wistar rats in other groups before administration on D1 are decreased significantly (P < 0.001), proving that the mouse TAO model is successfully established in each group.

Compared with the case before administration on D1 in each group, after the last administration on D23, the concentrations of the TSH in the serums of the Wistar rats in the S-ASODN-1 high-dose group and the positive drug group are increased significantly (P < 0.001 and P < 0.05). The concentration of the TSH is increased to 1.21-2.00 IU/mL, and the increase is about 7 to 16 times that of the model group, exhibiting a good therapeutic effect. The increase in the concentration of the TSH of the positive drug 2 group is 7.67 times that of the model group. The concentration of the TSH of the S-ASODN-1 high-dose group is increased to 2.00 IU/mL after the last administration on D23, and the increase is 16.11 times that of the model group and 2.10 times that of the positive drug 2 group, exhibiting the most excellent therapeutic effect.

As can be seen from the above data, an order of therapeutic effects of each group is S-ASODN-1 high-dose group > positive drug 2 group.

### 4.3.4 IGF-1R detection

The results of detecting the concentrations of the IGF-1Rs in the intact OD tissues on D23 are shown in the following table and FIG. 6-4:

**Table 12**

| Group | IGF-1R Concentration (ng/mL) |
|---|---|
| | D23 |
| control group | 0.68±0.12 |
| model group | 2.69±0.17 |
| S-ASODN-1 high-dose group | 0.65±0.13 |
| positive drug 2 group | 1.17±0.10 |

During the experiment, compared with the control group, the concentrations of the IGF-1Rs in the OD tissues of the Wistar rats in the model group and the positive drug 2 group are increased significantly (P < 0.001 and P < 0.01), and no statistical difference exists in the concentration of the IGF-1R in the OD tissues of the Wistar rats in the S-ASODN-1 high-dose group (P > 0.05).

Compared with the model group, the concentrations of the IGF-1Rs in the OD tissues of the Wistar rats in the positive drug group and the S-ASODN-1 high-dose group are decreased significantly (P < 0.001).

The above are merely preferred implementation modes of the present application. It is to be noted that for those skilled in the art, a number of improvements and modifications may be made without departing from the principle of the present application, and these improvements and modifications are within the scope of the present application.

## Claims

1. A therapeutically effective amount of a thioantisense oligonucleotide targeting an IGF-1R gene or a composition thereof for use in preparation of a drug for treating a disease caused by an abnormal thyroid function, wherein the thioantisense oligonucleotide targeting the IGF-1R gene has:
(I) a nucleotide sequence shown in SEQ ID No. 1; or
(II) a nucleotide sequence obtained after one or more nucleotide sequences in the nucleotide sequence shown in (I) are substituted or deleted or one or more nucleotide sequences are added to the nucleotide sequence shown in (I) and having a same or similar function as the nucleotide sequence shown in (I); or
(III) a nucleotide sequence having at least 80% nucleotide identity to the nucleotide sequence shown in (I) or (II);
preferably, the thioantisense oligonucleotide targeting the IGF-1R gene has a nucleotide sequence having 85%, 90%, 95%, 96%, 97%, 98%, 99% or more nucleotide identity to the nucleotide sequence shown in (I) or (II).

2. The therapeutically effective amount of a thioantisense oligonucleotide targeting an IGF-1R gene or a composition thereof according to claim 1, wherein the disease caused by the abnormal thyroid function comprises thyroid-associated ophthalmopathy.

3. The therapeutically effective amount of a thioantisense oligonucleotide targeting an IGF-1R gene or a composition thereof according to claim 1, wherein the thioantisense oligonucleotide targeting the IGF-1R gene further has other chemical modifications.

4. The therapeutically effective amount of a thioantisense oligonucleotide targeting an IGF-1R gene or a composition thereof according to any one of claims 1 to 3, wherein the other chemical modifications are selected from one or more of a locked nucleic acid modification, a methoxyethyl modification at a 2 position and an oxomethyl modification at a 2 position.

5. The therapeutically effective amount of a thioantisense oligonucleotide targeting an IGF-1R gene or a composition thereof according to any one of claims 1 to 4, wherein the composition further comprises at least one additional active agent.

6. The therapeutically effective amount of a thioantisense oligonucleotide targeting an IGF-1R gene or a composition thereof according to claim 5, wherein the at least one additional active agent is a therapeutic agent.

7. The therapeutically effective amount of a thioantisense oligonucleotide targeting an IGF-1R gene or a composition thereof according to claim 6, wherein the therapeutic agent is selected from one or more of a corticosteroid, a biological agent and a traditional immunosuppressant.

8. The therapeutically effective amount of a thioantisense oligonucleotide targeting an IGF-1R gene or a composition thereof according to claim 7, wherein the corticosteroid is selected from a glucocorticoid.

9. The therapeutically effective amount of a thioantisense oligonucleotide targeting an IGF-1R gene or a composition thereof according to claim 7, wherein the biological agent is selected from one or more of a CD20+B cell inhibitor, an IL-6R antibody, an IL-17A antagonist, an FcRn antagonist, an IL-11R blocking antibody, an anti-TNFα antibody and a thyroid-stimulating hormone receptor (TSHR) inhibitor.

10. The therapeutically effective amount of a thioantisense oligonucleotide targeting an IGF-1R gene or a composition thereof according to claim 7, wherein the traditional immunosuppressant is selected from one or more of mycophenolate mofetil, ciclosporin, methotrexate and azathioprine.

11. The therapeutically effective amount of a thioantisense oligonucleotide targeting an IGF-1R gene or a composition thereof according to claim 6 or 7, wherein the therapeutic agent is selected from one or more of secukinumab, tocilizumab, satralizumab, vunakizumab, batoclimab, TOUR006, LASN01, infliximab, rituximab and selenium.

12. The therapeutically effective amount of a thioantisense oligonucleotide targeting an IGF-1R gene or a composition thereof according to any one of claims 2 to 11, wherein the thyroid-associated ophthalmopathy manifests as one or more of the following symptoms: eyelid syndrome, exophthalmos, diplopia, ocular motility disorder, chemosis, optic neuropathy, keratopathy, conjunctival lesion and keratopathy and systemic symptoms of patients accompanied by hyperthyroidism;
preferably, the diplopia comprises constant diplopia, non-constant diplopia and intermittent diplopia.

13. The therapeutically effective amount of a thioantisense oligonucleotide targeting an IGF-1R gene or a composition thereof according to any one of claims 1 to 12, wherein the thioantisense oligonucleotide targeting the IGF-1R gene or the composition thereof is formulated as a lyophilized agent or an injection.

14. The therapeutically effective amount of a thioantisense oligonucleotide targeting an IGF-1R gene or a composition thereof according to any one of claims 1 to 13, wherein the thioantisense oligonucleotide targeting the IGF-1R gene or the composition thereof is administered in combination with one or more thyroid-associated ophthalmopathy treatments;
preferably, the one or more thyroid-associated ophthalmopathy treatments are radiotherapy or/and surgical treatments.

15. The therapeutically effective amount of a thioantisense oligonucleotide targeting an IGF-1R gene or a composition thereof according to any one of claims 1 to 14, wherein a dose of the thioantisense oligonucleotide targeting the IGF-1R gene comprises 2.5-10 mg/kg.
